# EUROPEAN PATENT APPLICATION

(11) **EP 4 394 420 A1**
(43) Date of publication of application: **03.07.2024**
(21) Application number: 23220401.6
(22) Date of filing: 27.12.2023
(51) Int. Cl.: G01R 33/28, A61B 34/00, A61B 90/00, A61B 5/055

(54) **INTEGRATED EXCESSIVE GAUSS NOTIFICATION FOR MRI BREAST BIOPSY SYSTEM**

(30) Priority: 28.12.2022 US 202263435614 P
(71) Applicant: Devicor Medical Products, Inc., Cincinnati, OH 45241 (US)
(72) Inventor: WAGNER, Kyle, Mason, Ohio 45040 (US); SHADIX, Peter, Cincinnati, Ohio 45209 (US); WAGNER, Gary S., Independence, Kentucky 41051 (US)
(74) Representative: Simmons & Simmons

(57) **Abstract**

A control module for use in an MRI guided biopsy procedure using an MRI coil includes a body, and a gauss détection assembly. The body includes one or more ports and a display. The ports are configured to couple the control module to a biopsy device. The display is configured to output one or more biopsy device status indicators. The gauss détection assembly includes one or more sensors integrated into the body of the control module. Each sensor of the one or more sensors is configured to detect an electromagnetic field emitted from the MRI coil.

## Description

### PRIORITY

This application claims priority to U.S. Provisional Patent App. No. 63/435,614, entitled "Integrated Excessive Gauss Notification for MRI Breast Biopsy System," filed on December 28, 2022, the disclosure of which is hereby incorporated by reference herein.

### BACKGROUND

Biopsy samples have been obtained in a variety of ways in various medical procedures using a variety of devices. Biopsy devices may be used under stereotactic guidance, ultrasound guidance, Magnetic Resonance Imaging (MRI) guidance, Positron Emission Mammography (PEM) guidance, Breast-Specific Gamma Imaging (BSGI) guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device.

Examples of such biopsy devices and biopsy system components are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pat. No. 7,442,171, entitled "Remote Thumbwheel for a Surgical Biopsy Device," issued October 8, 2008; U.S. Pat. No. 7,854,706, entitled "Clutch and Valving System for Tetherless Biopsy Device," issued December 1, 2010; U.S. Pat. No. 7,938,786, entitled "Vacuum Timing Algorithm for Biopsy Device," issued May 10, 2011; and U.S. Pat. No. 8,118,755, entitled "Biopsy Sample Storage," issued February 21, 2012; and U.S. Pat. No. 10,201,333, entitled "MRI Biopsy System," issued February 12, 2019. The disclosure of each of the above-cited U.S. Patents is incorporated by reference herein.

MRI guided biopsy procedures may involve unique operational constraints due to the strong magnetic field associated with the area proximate the MRI coil. The presence of this strong magnetic field may present particular constraints with respect to ferromagnetic objects because such objects may be attracted to the strong magnetic field. Additionally, operation of one or more components including ferromagnetic materials may be subject to operational interference, damage, and/or degradation due to the strong magnetic field. Thus, certain components may be isolated or otherwise segregated from the MRI coil during an MRI guided biopsy procedure.

Isolation or segregation of components from the MRI coil may introduce usability challenges such as trip hazards, patient interaction challenges, and logistical challenges from moving between various components associated with the procedure. Thus, in some circumstances it may be desirable to move certain components closer to the MRI coil. Although some proximity of ferromagnetic objects relative to the MRI coil may be tolerated, the strength of the magnetic field and the specific distance separating the MRI coil from the ferromagnetic objects are factors that directly influence this tolerance.

Some components may be used to minimize the challenges associated with MRI guided biopsy procedures. For instance, gauss alarms may be used to provide feedback when ferromagnetic objects are moved too close to the MRI coil. An example of such a gauss alarm is disclosed in U.S. Pat. No. 8,378,836, entitled "Magnetic Field Strength Threshold Alarm," issued on February 19, 2013. However, such gauss alarms have been configured as separate components that may be clipped or otherwise secured to a ferromagnetic object. This separate configuration may be undesirable because the placement of the gauss alarm may be only relative to the ferromagnetic object generally, not particular concentrations of ferromagnetic components contained within the object. Furthermore, the placement of the gauss alarm may be limited to a particular region of the ferromagnetic object. With larger objects, such as a control module, gauss alarm positioning may result in differences in placement of the object relative to the MRI coil due to the particular orientation of the object relative to the MRI coil. Accordingly, it may be desirable to use other gauss detection and/or fixation configurations in connection with MRI guided breast biopsy procedures.

While several systems and methods have been made and used for obtaining a biopsy sample, it is believed that no one prior to the inventor has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an example of an MRI biopsy system including a biopsy device and a control module;
FIG. 2 depicts a perspective view of an MRI coil for use with the MRI biopsy system of FIG. 1;
FIG. 3 depicts a top plan view of an MRI suite for use with the MRI biopsy system of FIG. 1;
FIG. 4 depicts a perspective view of an alternative example of a control module for use with the MRI biopsy system of FIG. 1;
FIG. 5 depicts a schematic view of a signal processing system for use with the control module of FIG. 4;
FIG. 6 depicts a top plan view of an MRI suite for use with the MRI biopsy system of FIG. 1 and the control module of FIG. 4;
FIG. 7 depicts a perspective view of another alternative example of a control module for use with the MRI biopsy system of FIG. 1;
FIG. 8 depicts a perspective view of yet another alternative example of a control module for use with the MRI biopsy system of FIG. 1; and
FIG. 9 depicts a schematic view of a user interface screen for use with any one of the control modules of FIGS. 1, 4, and 8.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Overview of Example MRI Biopsy System

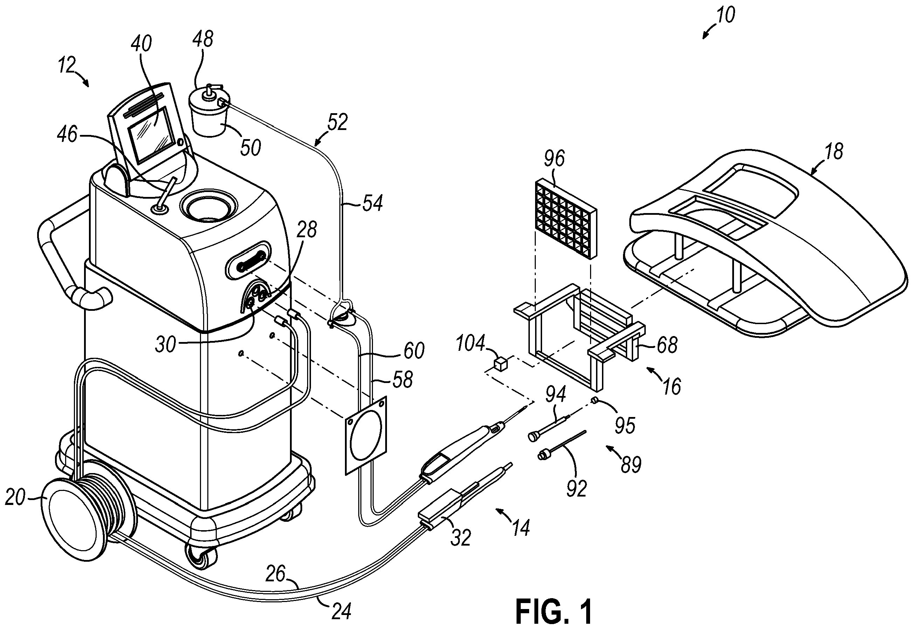

FIG. 1 shows an example of a Magnetic Resonance Imaging (MRI) compatible biopsy system (10) that may be used to collect one or more biopsy samples during an MRI guided biopsy procedure. MRI biopsy system (10) includes a control module (12) that may be placed outside of a shielded room containing an MRI coil to mitigate detrimental interaction with its strong magnetic field and/or sensitive radio frequency (RF) signal detection sensors and/or antennas. As described in U.S. Pat. No. 6,752,768, which is hereby incorporated by reference in its entirety, a range of preprogrammed functionality may be incorporated into control module (12) to assist in taking tissue samples.

MRI biopsy system (10) further includes a biopsy device (14), a localization fixture (16), and a patient support table (18). Control module (12) is generally configured to control and power biopsy device (14) during use with localization assembly (15). As will be understood, biopsy device (14) may be positioned and guided by localization fixture (16) attached to patient support table (18) that may be placed upon a gantry (not shown) of an MRI coil or other imaging machine.

In the present version, control module (12) may be mechanically, electrically, and/or pneumatically coupled to biopsy device (14) so that components may be segregated that need to be spaced away from the strong magnetic field and the sensitive RF receiving components of an MRI coil. Thus, in some versions, control module (12) may include a cable management spool (20) that may be configured to aid in management of various cables such as an electrical cable (24) and/or mechanical cable (26). Such cables (24, 26) may be configured to communicate control signals and cutter rotation/advancement motions respectively and may be connected to respective electrical and mechanical ports (28, 30) in control module (12). It should be understood that such components described above as being associated with control module (12) are merely optional.

Vacuum assist is provided by first vacuum line (46) that connects between control module (12) and outlet port (48) of vacuum canister (50) that catches liquid and solid debris. Tubing kit (52) completes the pneumatic communication between control module (12) and biopsy device (14). In particular, second vacuum line (54) is connected to inlet port (56) of vacuum canister (50). Second vacuum line (54) divides into two vacuum lines (58, 60) that are attached to biopsy device (14). In this configuration, control module (12) may communicate vacuum, saline, atmospheric air, and/or other fluids to various portions of biopsy device (14) for use in collecting one or more tissue samples.

Control module (12) may further include a display (40). Display (40) may be configured to control certain operational features of control module (12) and/or biopsy device (14). Display (40) may further be configured to output certain control module (12) and/or biopsy device (14) status indicators. By way of example only, some such status indicators may include the level of vacuum supplied to biopsy device (14), a particular mode of operation associated with biopsy device (14), the position of components within biopsy device (14) (e.g., needle, cutter, tissue sample holder, etc.), and/or etc. In some versions, display (40) may receive operator input directly with display (40) being configured as a touchscreen. In addition, or in the alternative, display (40) may be associated with one or more buttons to facilitate receipt of input.

Of course, the above-described control module (12) is merely one example. Any other suitable type of control module (12) and associated components may be used. In some versions, control module (12) may be configured in accordance with any one or more of the teachings of US Pat. No. 10,201,333, entitled "MRI Biopsy System," issued on February 12, 2019, the disclosure of which is incorporated by reference herein. In other versions, control module (12) may instead be configured and operable in accordance with the teachings of U.S. Pub. No. 2008/0228103, entitled "Vacuum Timing Algorithm for Biopsy Device," published September 18, 2008, the disclosure of which is incorporated by reference herein. In still other versions, control module (12) may instead be configured and operable in accordance with the teachings of U.S. Patent No. 8,328,732, entitled "Control Module Interface for MRI Biopsy Device," issued December 11, 2012, the disclosure of which is incorporated by reference herein. Alternatively, control module (12) may have any other suitable components, features, configurations, functionalities, operability, etc. Other suitable variations of control module (12) and associated components will be apparent to those of ordinary skill in the art in view of the teachings herein.

Localization fixture (16) includes a localization framework (68) and a grid plate (96). Localization framework (68) may include one or more guides, struts, pillars, and/or tracks. Such structures may be configured to removably hold grid plate (96) in position relative to patient support table (18). Furthermore, in some versions of localization framework (68) certain structures thereof may be adjustable to permit control over the particular positioning of grid plate (96) relative to patient support table (18). As will be described in greater detail below, the combination of localization framework (68) and grid plate (96) may be configured to receive one or more components associated with biopsy device (14) to control insertion of biopsy device (14) into a patient. Additionally, in some versions, structures of localization framework (68) and/or grid plate (96) may be used to manipulate portions of a patient (e.g., breast compression) to further facilitate insertion of biopsy device (14) into a patient.

It should be understood that the above-described localization fixture (16) is merely one example. Any other suitable type of localization fixture (16) or assembly may be used, including but not limited to localization fixtures (16) that use localization frame work (68), grid plate (96) and/or patient support table (18) different from those described above. Other suitable components, features, configurations, functionalities, operability, etc. for a localization fixture (16) will be apparent to those of ordinary skill in the art in view of the teachings herein.

As shown in FIG. 1, one version of biopsy device (14) may comprise holster portion (32) and probe (91). In some versions, holster portion (32) may be configured as a reusable component, while probe (91) may be configured as a disposable component. Thus, holster portion (32) may include components particularly suited for re-use such as drive components (e.g., gears, shafts, lead screws, etc.), control circuits, and/or etc. Additionally, holster portion (32) may be entirely sealed to permit re-use and associated sterilization procedures. Meanwhile, probe (91) may include components particularly suited for disposability. Such components may include, for example, components that have direct contact with tissue and/or bodily fluids such as fluid tubes, needles, cutting surfaces, tissue sample holders, and/or etc.

Probe (91) of the present example includes a needle (90) that may be used to penetrate a patient and collect one or more tissue samples. Although not shown, it should be understood that needle (90) may include an outer cannula defining a lateral aperture and a cutter movable relative to the outer cannula to sever tissue received within the lateral aperture. Thus, it should be understood that any one or more of probe (91) or holster portion (32) may include certain drive components to facilitate movement of needle (90) and/or other components associated with needle (90) such as the cutter.

By way of example only, biopsy device (14) may be configured in accordance with at least some of the teachings of U.S. Pub. No. 2010/0160824, the disclosure of which is incorporated by reference herein; U.S. Patent Pub. No. 2013/0144188, entitled "Biopsy Device with Slide-In Probe," published June 6, 2013, the disclosure of which is incorporated by reference herein; U.S. Patent Pub. No. 2013/0324882, entitled "Control for Biopsy Device," published December 5, 2013, the disclosure of which is incorporated by reference herein; U.S. Patent Pub. No. 2014/0039343, entitled "Biopsy System," published February 6, 2014, the disclosure of which is incorporated by reference herein; and/or U.S. Patent App. No. 14/469,761, entitled "Tissue Collection Assembly for Biopsy Device," filed August 27, 2014, the disclosure of which is incorporated by reference herein.

In some versions, MRI biopsy system (10) may further include a targeting set (89) to facilitate localization of needle (90) relative to a patient disposed on patient support table (18). In such versions, targeting set (89) may include a cannula (94), an obturator (92), a guide cube (104), and a depth stop (95). In such versions, guide cube (104) may be used in connection with grid plate (96) described above to provide the location of penetration relative to a patient. The combination of cannula (94) and obturator (92) may then be used for insertion through guide cube (104) and into a patient. During insertion thereof, the depth of insertion of cannula (94) and obturator (92) may be restricted using depth stop (95), which may stop distal progress of cannula (94) and obturator (92) through engagement with guide cube (104). Cannula (94) and obturator (92) may include limited or no ferromagnetic materials, so the particular position of cannula (94) and obturator (92) within a patient may be confirmed under MRI guidance. Once a desired position is established, obturator (92) may be removed from cannula (94) and needle (90) may be inserted in place of obturator (92) while the MRI coil is off and one or more tissue samples may be collected using needle (90).

Of course, the above-described targeting set (89) is merely one example. Any other suitable type of targeting set (89) and associated components may be used. In some versions, targeting set (89) may be configured in accordance with any one or more of the teachings of US Pat. No. 10,201,333, entitled "MRI Biopsy System," issued on February 12, 2019, the disclosure of which is incorporated by reference herein. In other versions, targeting set (89) may instead be configured and operable in accordance with the teachings of U.S. Pub. No. 2007/0255168, entitled "Grid and Rotatable Cube Guide Localization Fixture for Biopsy Device," published November 1, 2007, and incorporated by reference herein. Alternatively, targeting set (89) may have any other suitable components, features, configurations, functionalities, operability, etc. Other suitable variations of targeting set (89) and associated components will be apparent to those of ordinary skill in the art in view of the teachings herein.

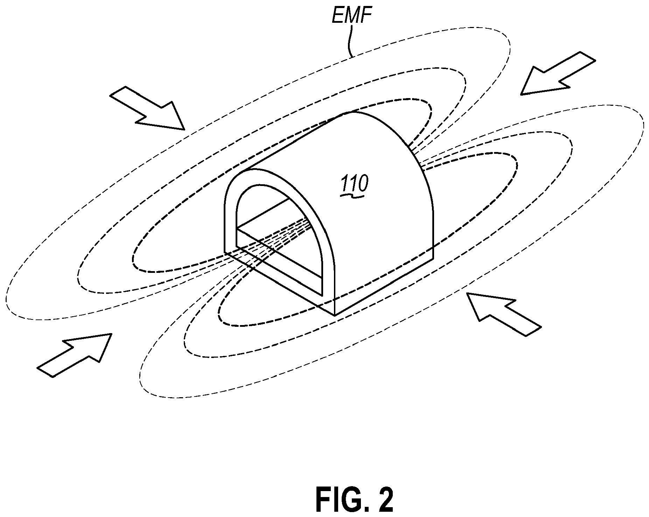

As described above, MRI biopsy system (10) may be used in connection with MRI guidance to facilitate collection of one or more tissue samples. An example of an MRI coil (110) that may be used in combination with MRI biopsy system (10) is shown in FIG. 2. As can be seen, MRI coil (110) may emit an electromagnetic field (EMF). Electromagnetic field (EMF) may be strongest in the area immediately adjacent to MRI coil (110) and weaken as the distance from MRI coil (110) increases. In other words, the strength of electromagnetic field (MF) may increase in the direction of the arrows visible in FIG. 2.

As a result of electromagnetic field (EMF) shown in FIG. 2, it may be undesirable to position ferromagnetic materials near MRI coil (110) where such ferromagnetic materials may substantially interact with electromagnetic field (EMF). For instance, in some circumstances, objects with sufficient concentrations of ferromagnetic materials may be drawn towards MRI coil (110) under the influence of electromagnetic field (EMF). In extreme instances, the force of electromagnetic field (EMF) may be strong enough to rapidly move certain objects with ferromagnetic materials toward MRI coil (110), turning such objects into projectiles. In other circumstances, objects may remain in position, but may interfere with signals generated by MRI coil (110), thus distorting imaging generated by MRI coil (110). In yet other circumstances, electrical devices containing ferromagnetic materials may malfunction, behave unpredictably, become damaged, or degrade in the presence of the electromagnetic field (EMF).

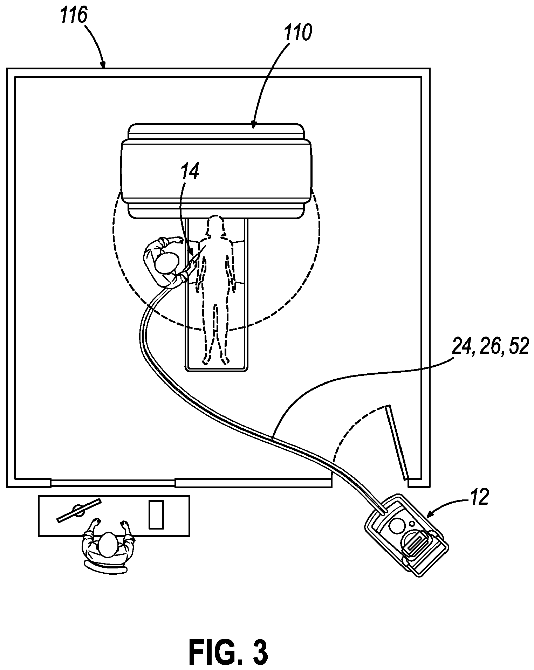

FIG. 3 shows a schematic representation of an example layout for MRI biopsy system (10) that may permit MRI guided biopsy procedures, while eliminating or reducing complications from interactions between electromagnetic field (EMF) and ferromagnetic components of MRI biopsy system (10). As can be seen, MRI coil (110) may be positioned within an MRI suite (116) or room. MRI suite (116) may include walls with electromagnetic shielding to isolate electromagnetic field (EMF) to a predetermined area.

As described above, control module (12) may include substantial ferromagnetic materials in the form of motors, electrical circuits, drive components, and/or etc. Thus, control module (12) may be positioned outside MRI suite (116) to isolate such components from electromagnetic field (EMF). Meanwhile, to permit operability of biopsy device (14), cables (24, 26) and/or tube kit (52) may extend from control module (12), into MRI suite (116) and to a region proximate MRI coil (110) where biopsy device (14) may be used to collect one or more tissue samples from a patient. In some versions, cables (24, 26) and tube kit (52) may extend for a length of 25 feet or more. Accordingly, it should be understood that MRI biopsy system (10) of the present version is generally configured to segregate many ferromagnetic components into control module (12) rather than biopsy device (14) so that such ferromagnetic components may be isolated from electromagnetic field (EMF) by positioning control module (12) outside of MRI suite (116).

### II. Example of Alternative Control Module with Integral Gauss Detection

In some circumstances, it may be desirable to include components similar to control module (12) within an MRI suite similar to MRI suite (116) described above. For instance, in the configuration of control module (12) and MRI suite (116) described above, the arrangement of control module (12) outside MRI suite (116) may lead to operational challenges in some circumstances. For instance, the remote nature of control module (12) relative to the clinician performing the biopsy procedure may contribute to challenges with making operational adjustments to control module (12), particularly mid-biopsy procedure. Additionally, under some circumstances, the length of cables (24, 26) and/or tube kit (52) may contribute to operational challenges. For instance, cables (24, 26) and/or tube kit (52) may introduce operational delays or inefficiencies when closing the door of MRI suite (116), which may require retraction of cables (24, 26) and/or tube kit (52). Cables (24, 26) and/or tube kit (52) may also present a trip hazard under some circumstances. The relatively long length of mechanical cable (26) may also lead to unintentional movement of mechanical cable (26) during a biopsy procedure due to internal torque within mechanical cable (26).

In view of the discussion above, it may be desirable under some circumstances to use a component similar to control module (12) within an MRI suite such as MRI suite (116). However, as noted above, the presence of an MRI coil such as MRI coil (110) may lead to undesirable effects due to the electromagnetic field generated by the MRI coil. Accordingly, it may be desirable to include certain features within a control module similar to control module (12) to permit the control module to be used within an MRI suite with limited interaction between the control module and the electromagnetic field generated by the MRI coil.

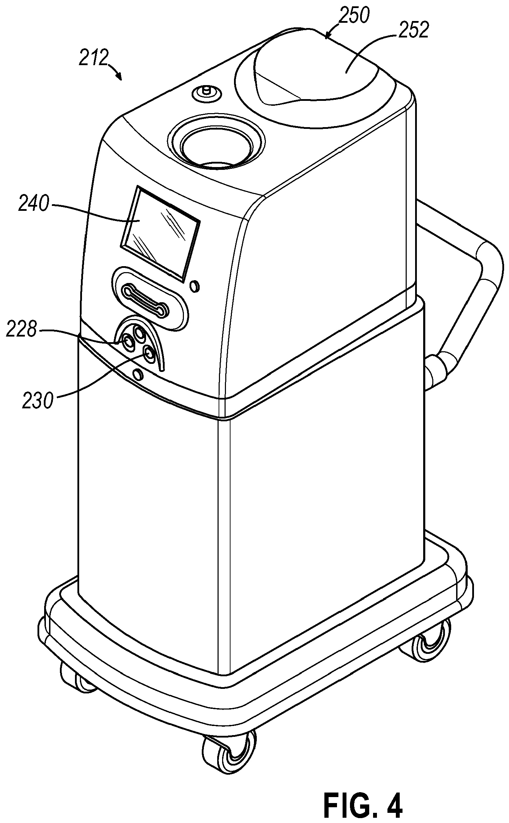

FIG. 4 shows an example of a control module (212) configured for use within an MRI suite similar to MRI suite (116) described above. Unless as otherwise described herein, control module (212) of the present version may be used with MRI biopsy system (10) as similarly described above with respect to control module (12) and in lieu of control module (12). Control module (212) of the present version is similar to control module (12) described above in that control module (212) is generally configured to control and power biopsy device (14) during use with localization assembly (15)

As similarly described above, control module (212) of the present version may be mechanically, electrically, and/or pneumatically coupled to biopsy device (14) so that components may be segregated that need to be spaced away from the strong magnetic field and the sensitive RF receiving components of an MRI coil. Thus, in some versions, control module (212) may include ports (228, 230), which may couple to cables (24, 26) as described above. Control module (212) may further be configured to receive vacuum canister (50) to connect tubing kit (52) thereto.

As with control module (12) described above, control module (212) of the present version may further include a display (240). As with display (40) described above, display (240) may be configured to control certain operational features of control module (12) and/or biopsy device (14) and communication operational status information associated with control module (12) and/or biopsy device (14). Additionally, as will be described in greater detail below, display (240) of the present version may be configured to interface with other components described herein to provide status information to an operator.

Unlike control module (12) described above, control module (212) of the present version includes a gauss detection assembly (250). Gauss detection assembly (250) is generally configured to detect electromagnetic radiation relative to control module (212) and provide an indication to an operator as to the particular level of electromagnetic radiation detected. As will be described in greater detail below, the detection provided by gauss detection assembly (250) may permit control module (212) to be positioned relatively close to an MRI coil, but yet far enough away so avoid unacceptable influence from the electromagnetic field generated by the MRI coil.

At least a portion of gauss detection assembly (250) is integrated into one or more structures of control module (212). In other words, one or more components of gauss detection assembly (250) may be permanently secured in a fixed position relative to a portion of control module (212). In this configuration, "permanently secured" refers to how one or more portions of gauss detection assembly (250) may be fixed or encased within a portion of control module (212) (e.g., the housing of control module (212) so that such components may not be readily be removed by a clinician. Of course, in some versions, "permanently secured" may also include some removability such as by a maintenance technician using one or more tools for removal of such components outside of clinical settings.

Gauss detection assembly (250) may include a detector array (252) configured to receive and detect electromagnetic radiation. As can be seen in FIG. 4, detector array (252) may be positioned in a fixed position on control module (212) proximate the top or highest point of control module (212). The top of control module (212) referred to herein may refer to the portion of control module opposite the wheels of control module (212) (where used) or the base of control module (212). In some versions, detector array (252) may be positioned within a top portion of control module (212). The term top portion of control module (212) in some circumstances may refer to the upper ½ of control module (212). In other circumstances, the term may refer to the upper % or upper 1/8 of control module (212).

As will be understood, the fixed position of detector array (252) may be desirable to permit all detected electromagnetic radiation to be from a fixed reference point. By having a fixed reference point, gauss detection assembly (250) may be precisely calibrated for the specific size and shape of control module (212). Additionally, the relatively high positioning of detector array (252) may be desirable so that detection of electromagnetic radiation occurs where the electromagnetic field will be the highest (e.g., the highest point of control module (212)) rather than the lowest (e.g., the bottom of control module (212)). Although detector array (252) is positioned in a relatively high position in the present example, lower positioning of detector array (252) is used in other examples. In such examples, adjustments to calibration of detector array (252) or software protocols are made to compensate for the lower gauss levels associated with the lower positioning.

In the present version, detector array (252) may include an optional protective cover. Such a protective cover may be desirable to protect potions of detector array (252) from incidental contact with biopsy procedure components, fluids, and/or etc. Such a protective cover may also facilitate efficient cleaning of the outer surface of control module (212) because one or more portions of detector array (252) may define an irregular surface. Of course, the protective cover is merely optional and may be omitted in some versions.

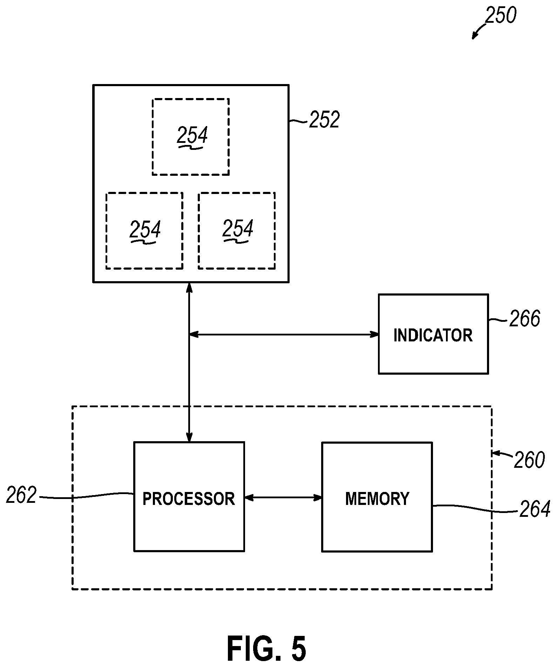

As best seen in FIG. 5, detector array (252) may include one or more sensors (254) disposed on a surface of detector array (252). Each sensor (254) of the one or more sensors (254) may include a hall effect sensor or other suitable sensor configured to detect electromagnetic radiation and/or an electromagnetic field. In particular, each sensor (254) may be configured to detect the presence and magnitude of an electromagnetic field and generate an output voltage. The output voltage may be processed by one or more processors to generate a digital signal, which can be directly proportional to the strength of the electromagnetic field. As will be described in greater detail below, this digital signal may be used in combination with threshold values to identify certain operational conditions during a biopsy procedure.

Each sensor (254) of the one or more sensors (254) is generally integrated or otherwise permanently secured relative to a portion of control module (212). As will be described in greater detail below, this integration or fixation of each sensor (254) may be desirable so that each sensor (254) may be correlated with control module (212) to account for the position of control module (212) relative to the electromagnetic field. In some versions, each sensor (254) may be encased within a portion of a housing of control module (212) so that each sensor (254) may be held in position. In other versions, each sensor (254) may be fastened to a portion of control module (212) by mechanical or adhesive fastening systems. Although one or more sensors (254) are described herein with the term "sensor," it should be understood that reference to one or more sensors (254) herein may encompass the presence of other components such as one or more sensors (254) being integrated into an integrated circuit chip on a printed circuit board (PCB).

As noted above, detector array (252) may include any suitable number of sensors (254) including one sensor (254), two sensors (254), three sensors (254), or more. Certain factors may make some sensor (254) configurations more desirable than other configurations. Some factors may include, for example, the specific shape of control module (212) or the desired practical use for control module (212) during a biopsy procedure. For instance, in single sensor (254) configurations, detector array (252) may detect the presence of an electromagnetic field at a particular point on control module (212). This configuration may be desirable in circumstances where control module (212) is of a generally symmetrical configuration and detector array (252) is positioned at the upper center of control module (212). This configuration may also be desirable in circumstances where control module (212) need not be positioned particularly close to an MRI coil.

In other configurations, the use of multiple sensors (254) may be desirable. For instance, where control module (212) is of an asymmetrical shape, or has other instruments or structures projecting therefrom, use of a single sensor (254) may be less desirable because detection of an electromagnetic field at one point may not correlate well to all parts of control module (212). Thus, in such circumstances, multiple sensors (254) may be used to provide detection of an electromagnetic field across multiple dimensions. In other words, the use of multiple sensors (254) may be desirable to provide greater resolution to the detection capabilities of detector array (252).

In merely one example of a multiple sensor (254) configuration, two sensors (254) may be used with each sensor (254) being positioned opposite of each other along a sensor axis. Although this sensor axis may be aligned with any particular structure of control module (212), in one configuration, the sensor axis may be aligned with a width axis of control module (212) extending from one side of control module (212) to another across the widest dimension of control module (212). In this configuration, each sensor (254) may be correlated with the widest sides of control module (212) to provide electromagnetic field detection corresponding to the widest parts of control module (212).

In another example of a multiple sensor (254) configuration, three sensors (254) may be used with each sensor (254) being positioned in a triangular configuration. In such a triangular configuration, two sensors (254) may be positioned along a sensor axis similar to the sensor axis described above with respect to the two sensor (254) configuration. Meanwhile, the third sensor (254) may be offset relative to the sensor axis toward a particular geometric feature of control module (212) (e.g., the front or rear of control module (212)). In this configuration, the addition of the third sensor (254) may be configured to provide additional resolution to detection of the electromagnetic field (e.g., about the X and Y axes). Additionally, in some configurations one or more of the three sensors (254) can be positioned along another axis in addition to the axes described above to provide further resolution to detection of the electromagnetic field (e.g., about the X, Y, and Z axes).

In yet another example of a multiple sensor (254) configuration, four sensors (254) may be used with each sensor (254) being positioned in a square or grid-shaped pattern. In such a square configuration, each sensor (254) may be aligned with a particular geometric feature of control module (212) such as each corner. Thus, each sensor (254) may be correlated with a given feature of control module (212) to provide additional resolution for detection of the electromagnetic field.

In the various multiple sensor (254) configurations described above, data from each individual sensor (254) can be combined to compare the data to certain predetermined threshold values. Such comparisons may be desirable to determine the existence of acceptable or unacceptable gauss levels. By way of example only, in one configuration, data is collected from each individual sensor (254) and then the highest gauss level between all the sensors is identified and then the highest level is used for comparison relative to the predetermined threshold values. In other examples, the data from all sensors can be combined to form a single value, which can then be compared to the predetermined threshold values. A variety of method of combining data can be used. For instance, in one method, the following formula is used to calculate a composite value: C = √(a²+b²+c²), where C represents the output composite value and letters a, b, and c represent the output values generated by each sensor. Of course various alternative, methods for combining data of sensors (254) can be used as will be apparent to those of ordinary skill in the art in view of the teachings herein.

Although several specific sensor (254) configurations are described herein, it should be understood that other suitable configuration may be used in other versions. In other suitable configurations, additional sensors (254) beyond the one, two, three, and four sensor (254) configurations described herein may be used. Additionally, although multiple sensor (254) configurations are described herein with respect to each sensor (254) being positioned on a common plane, it should be understood that in other versions, one or more sensors (254) may be positioned on different planes relative to other sensors (254). Such multi-planar sensor (254) configurations may be configured to provide detection of the electromagnetic field across different dimensions relative to single plane configurations.

Gauss detection assembly (250) further includes a signal processing system (260) in communication with detector array (252) to receive and process signals generated by one or more sensors (254). Although now shown, it should be understood that in some versions, one or more portions of signal processing system (260) may be in communication with other portions of control module (212). In such configurations, signal processing system (260) may also be configured to implement various functions of control module (212) and/or biopsy device (14).

Signal processing system (260) includes a data processor (262), a memory (264), and an indicator (266). Data processor (262) is in communication with detector array (252) to receive signals from one or more sensors (254) of detector array (252). Data processor (262) is further in communication with memory (264), which may be used in combination with data processor (262) to facilitate various functions of data processor (262) described in greater detail below. Although data processor (262) of the present version is shown as a single element, it should be understood that data processor (262) may include multiple processors in some examples, with one or more of such multiple processors being dedicated to particular functions.

Memory (264) may include random access memory (RAM), which may be configured for short-term storage of data. Additionally, or in the alternative, memory (264) may further include a solid-state drive or a hard disk drive, which may be configured for long-term storage of data. In versions where memory (264) includes both short-term and long-term storage of data, such short-term and long-term storage elements may be in communication with each other to facilitate transfer of data between short-term storage and long-term storage. Together, data processor (262) and memory (264) may be configured to execute computer programs, in the form of software, to implement various functions of biopsy device (14), control module (212), and/or gauss detection assembly (250) as described in greater detail below.

In some versions, data processor (262) and memory (264) may be disposed within control module (212) and may communicate with other elements of MRI biopsy system (10) via cable (24). In other versions, data processor (262) and/or memory (264) may be disposed within other components such as one or more portions of biopsy device (14). Such a configuration may be particularly desirable where data processor (262) is divided into multiple separate data processors (262) or sub-processors. In such examples, one or more data processors (262) may be disposed within one or more portions of biopsy device (14), while one or more data processors (262) may be disposed within control module (212). In such configurations, specific data processors (262) may be localized in connection with particular functions. In yet other versions, data processor (262) and/or memory (264) may be disposed entirely remotely relative to MRI biopsy system (10) to permit the processing of data elsewhere (e.g., cloud-based solutions).

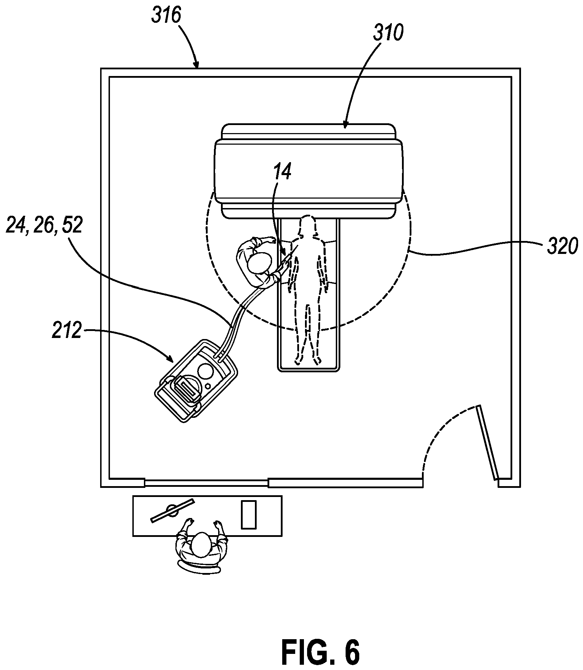

FIG. 6 shows a schematic representation of an example layout for MRI biopsy system (10) in combination with control module (212). The layout shown in FIG. 6 is similar to the layout shown in FIG. 3 in that the layout may permit MRI guided biopsy procedures, while eliminating or reducing complications from interactions between electromagnetic field (EMF) and ferromagnetic components of MRI biopsy system (10). However, unlike the layout described above with respect to FIG. 3, the present layout may be configured for enhanced use by control module (212) being proximate a clinician, as will be described in greater detail below.

As can be seen, an MRI coil (310) may similarly be positioned within an MRI suite (316) or room. MRI suite (316) may include walls with electromagnetic shielding to isolate electromagnetic field (EMF) to a predetermined area. Although control module (212) of the present version may still include substantial ferromagnetic materials in the form of motors, electrical circuits, drive components, and/or etc., control module (212) may nonetheless still be positioned within MRI suite (316). Specifically, gauss detection assembly (250) may permit control module (212) to be positioned far enough from MRI coil (310) to effectively isolate such components from electromagnetic field (EMF); yet control module (212) may also remain close enough to enhance easy of operation with respect to biopsy device (14), cables (24, 26) and/or tube kit (52). In other words, all of control module (212), biopsy device (14), cables (24, 26) and tube kit (52) be positioned within MRI suite (316) within a region proximate MRI coil (310) where biopsy device (14) may be used to collect one or more tissue samples from a patient. Consequently, cables (24, 26) and tube kit (52) in the present configuration may have a reduced length of 10 feet or less. Alternatively, the same length may still be used, but portions of cables (24, 26) and/or tube kit (52) may be stowed to shorten the effective length of cables (24, 26) and/or tube kit (52).

In the configuration described above, gauss detection assembly (250) may be used in a variety of ways to facilitate use of control module (212) in relatively close proximity with MRI coil (310). For instance, gauss detection assembly (250) may be generally configured to provide one or more operational controls to avoid control module (212) crossing into a predetermined gauss zone (320). As can be seen, gauss zone (320) may be in the form of a generally arcuate line extending from one side of MRI coil (310) to another. Although gauss zone (320) is illustrated two dimensionally as an arc shaped line, it should be understood that in practice, gauss zone (320) defines a generally semispherical shape in three dimensions. Regardless, such a line (or semi-sphere in three dimensions) is generally an imaginary line, which may represent the point where proximity to MRI coil (310) is too close for optimal operation of control module (212). In some versions, gauss zone (320) may also be physically present in the form of lines on the floor of MRI suite (316). Although a physical manifestation of gauss zone (320) is merely optional. Although a single gauss zone (320) is illustrated in the present version, it should be understood that multiple gauss zones (320) may be used to represent different levels of electromagnetic field (EMF) strength. For instance, in some versions, three gauss zones (320) may be used with one gauss zone (320) representing optimal operations of control module (212), another gauss zone (320) representing suboptimal operations of control module (212), and another gauss zone (320) representing regions where control module (212) and MRI coil (310) are unable to be operated simultaneously.

Gauss detection assembly (250) may be configured to detect the position of control module (212) relative to gauss zone (320) and to initiate one or more operational controls at various positions of control module (212) relative to gauss zone (320). In particular, detector array (252) may be in communication with signal processing system (260) and signal processing system (260) may continuously monitor the strength of the electromagnetic field (EMF) detected by director array (252). Signal processing system (260) may also compare the detected strength of the electromagnetic field (EMF) to certain predetermined thresholds. When signal processing system (260) detects the detected strength of the electromagnetic field (EMF) crossing the one or more predetermined thresholds, signal processing system (260) may initiate one ore more operational controls discussed in greater detail below. In some versions, signal processing system (260) may be calibrated to account for the dimensions of control module (212) relative to the detected strength of the electromagnetic field (EMF) and gauss zone (320) so that there may be correspondence between the physical position of control module (212), the detected strength of the electromagnetic field (EMF), and gauss zone (320).

In versions where detector array (252) includes a single sensor (254), signal processing system (260) may use relatively simple comparisons and/or computations to determine the position of control module (212) relative to gauss zone (320). In versions where multiple sensors (254) are used, signal processing system (260) may use more complex algorithums to determine the position of control module (212) relative to gauss zone (320). For instance, in some versions, signal processing system (260) may receive the detected strength of the electromagnetic field (EMF) from multiple sensors (254) simultaneously. Signal processing system (260) may then use the simultaneous input from multiple sensors (254) to compute or triangulate the position of control module (212) relative to gauss zone (320) in three-dimensional space. Signal processing system (260) may then compare this positioning to certain predetermined threshold values, while also making adjustments for specific geometric features of control module (212). Based on this comparison, signal processing system (260) may then determine whether to initiate one or more operational controls as discussed in further detail below.

Regardless of the process used to process data from detector array (252), crossing a predetermined threshold may initiate one or more operational controls. Such operational controls may include a variety of operations configured to operate in real-time and generally configured to draw an operator's attention to the position of control module (212) relative to MRI coil (310). For instance, in one aspect of an operational control, indicator (266) may provide visual feedback, auditory feedback, tactile feedback, or a combination thereof to alert an operator that at least a portion of control module (212) has crossed gauss zone (320). More specifically, indicator (266) may illuminate a light of a particular color (e.g., red, yellow, etc.), generate an auditory alarm (e.g., via piezoelectric buzzer), and/or generate a vibration in one or more components MRI biopsy system (10) such as biopsy device (14). In some versions, indicator (266) may be positioned on one or more portions of control module (212), biopsy device (14), other components of biopsy system (10), or as a stand-alone component. In other versions, indicator (266) may be incorporated into display (240) as a part of a graphical user interface.

In some versions, it may be desirable to provide an operational control with greater detail rather than the binary output operational controls described above. For instance, in some versions, indicator (266) may include a plurality of lights of varying colors to indicate different control module (212) position information. Specifically, indicator (266) may include a stop light configuration. In some such stop light configurations, indicator (266) may be configured with a green, yellow, and red light or be otherwise configured to emit such colors. In such configurations, green can indicate control module (212) is positioned outside gauss zone (320), yellow can indicate control module (212) is positioned near gauss zone (320), and red can indicate control module (212) is positioned inside gauss zone (320). In another stop light configuration, the red light may be omitted or otherwise not used and indicator (266) may be configured to emit only a green and yellow light. In such configurations, the green light can flash at a frequency similar to a heartbeat to indicate control module (212) being positioned outside gauss zone (320). Meanwhile, the yellow light may solidly illuminate to indicate control module (212) being positioned inside gauss zone (320). In still other examples, a single light can be used and illuminated (e.g., red or yellow light) only when control module (212) is positoined inside gauss zone (320). In such examples, the use of a single light or other indicator may be desirable to simplify use, avoid confusion with other alarms, and/or improve operational efficency. Similar feedback systems may be provided by indicator (266) in auditory and/or tactile form either in-lieu of the visual stop light configurations or in combination with the visual stop light configurations. In such auditory and/or tactile forms, different sounds or vibrations indicating different positions of control module (212) relative to gauss zone (320) may be used. In still other versions, the visual stop light configuration of indicator (266) may be used in combination with a buzzer such as a piezoelectric buzzer. In such a configuration, the visual stop light configuration of indicator (266) may be used to provide real-time status information, while the buzzer may be used to indicate when control module (212) has crossed a predetermined threshold (e.g., into gauss zone (320)).

In addition, or in the alternative, such stop light configurations may be integrated into display (240) in combination with a graphical user interface. In still other versions, display (240) may include a schematic representation of control module (212) and MRI coil (310) to graphically illustrate the position of control module (212) relative to MRI coil (310). In such versions, display (240) may show a prompt, dialog box, alert icon, or other graphical feature when control module (212) is moved into a predetermined position relative to MRI coil (310).

Operational controls used herein may also include controls that effect the physical operation of MRI biopsy system (10). For instance, signal processing system (260) may be configured to initiate certain functional lockouts to disable one or more functions of MRI biopsy system (10). One such functional lockouts may include disabling biopsy device (14) to prevent collection of tissue samples. Another such functional lockout may include initiating wheel locks on control module (212) to prevent movement of control module (212).

Although several aspects of operational controls are described herein, it should be understood that various suitable combinations of operational controls may be used. For instance, one or more functions described above with respect to indicator (266) may be combined with one or more functional lockouts. In other versions, multiple indicators (266) may be used in combination to provide multiple modes of operational controls and/or multiple operational control sources/locations.

### III. Example of Alternative Control Module with Task Light

In some instances, MRI suites such as MRI suites (116, 316) may have suboptimal ambient lighting for the purposes of breast biopsy procedures performed using MRI biopsy system (10). For instance, MRI suites may be configured to perform a variety of medical procedures. Frequently, lighting in such medical procedures is not critical because substantial clinician-patient interaction may not be needed in the region proximate the MRI coil. By contrast, in an MRI guided breast biopsy procedure performed with MRI biopsy system (10), substantial clinician-patient interaction may occur in the region proximate the MRI coil. Specifically, the use of localization fixture (16), patient support table (18), localization framework (68), targeting set (89), and/or etc. may result in multiple steps being performed in the region proximate the MRI coil.

During use of use of localization fixture (16), patient support table (18), localization framework (68), targeting set (89), and/or etc. it the region proximate the MRI coil, lighting of the work area near such components may be suboptimal. For instance, overhead lighting may vary from one MRI suite to another. Additionally, the MRI coil and components of MRI biopsy system (10) may cast shadows over the work area. As a result of these factors, procedural steps involving components such as localization fixture (16), patient support table (18), localization framework (68), targeting set (89), and/or etc. may be cumbersome, inefficient, or otherwise challenging. Accordingly, it may be desirable to incorporate features into MRI biopsy system (10) to provide consistent lighting from one MRI suite to another.

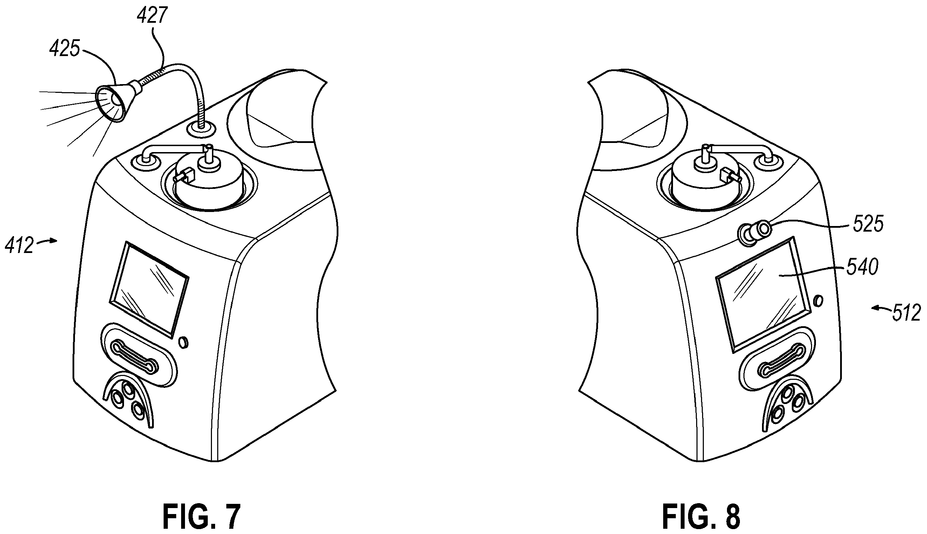

FIGS. 7 through 9 show aspects of a control module such as control modules (12, 212) described above that include features configured to improve lighting during an MRI guided biopsy procedure. For instance, FIG. 7 shows an example of a control module (412) that is generally configured to illuminate work areas during an MRI guided breast biopsy procedure. Unless as otherwise described herein, control module (412) of the present version may be used with MRI biopsy system (10) as similarly described above with respect to control module (12) and in lieu of control module (12). Control module (412) of the present version is substantially similar to control modules (12, 212) described above in that control module (412) is generally configured to control and power biopsy device (14) during use with localization assembly (15). Additionally, although not shown, it should be understood that control module (412) may also optionally include gauss detection assemblies such as gauss detection assembly (250) described above.

Unlike control modules (12, 212) described above, control module (412) of the present version includes a task light (425) secured to a portion of control module (412) and extending from an outer housing of control module (412). Task light (425) is generally configured to provide light that may be movably directed towards areas proximate control module (412). The light provided may be generally unfocused or diffused to provide uniform lighting to an area. In other versions, task light (425) may be configured to adjust the focus of emitted light from a generally unfocused or diffused configuration to a bright and focused configuration. In such versions, adjustment of focus may be desirable to switch between illuminating a large area consistently and illuminating a small area with bright and focused light.

Task light (425) of the present version includes a goose neck (427) that may extend from a light source of task light (425) to control module (412). Goose neck (427) is generally configured to provide spatial adjustment of the light source of task light (425) form one region to another. In the present version, goose neck (427) is a semi-rigid structure that may permit intentional bending thereof, but may remain static once bent. In other versions, goose neck (427) may take on a variety of alternative forms, either in addition to or in lieu of the semi-rigid structure of goose neck (427). Suitable additional or alternative features of goose neck (427) may include features such as one or more hinged support arms, one or more panel swivel mounts, and/or etc. Additionally, directionality of task light (425) may be provided by moving control module (412) itself.

Task light (425) is generally secured proximate an upper portion of control module (412). Such a positioning may be desirable in some versions to reduce upward shadows on the illuminated area. Additionally, the presence of goose neck (427) or other features may further increase the height of task light (425) relative to control module (412) to further reduce the presence of upward shadows. In still other versions, multiple task lights (425) may be used to provide lighting from varying angles.

Task light (425) may include a switch so that an operator may manually turn task light (425) off and on. In other versions, task light (425) may include an automatic on/off functionality. For instance, in some versions, power for task light (425) may be integrated with internal control circuitry and processors of control module (412). In such versions, control circuitry and/or processors may switch off task light (425) when control module (412) is operated in modes where lighting is not requires such as sleep mode, imaging mode, and/or etc.

FIG. 8 shows another control module (512) that is generally configured to illuminate work areas during an MRI guided breast biopsy procedure. Unless as otherwise described herein, control module (512) of the present version may be used with MRI biopsy system (10) as similarly described above with respect to control module (12) and in lieu of control module (12). Control module (512) of the present version is substantially similar to control modules (12, 212) described above in that control module (512) is generally configured to control and power biopsy device (14) during use with localization assembly (15). Likewise, control module (512) of the present version, may include a display (540) to permit control of operation of biopsy device (14) via a graphical user interface. Additionally, although not shown, it should be understood that control module (512) may also optionally include gauss detection assemblies such as gauss detection assembly (250) described above.

Unlike control modules (12, 212) described above, control module (512) of the present version includes a task light (525) secured to a portion of control module (512) and extending from an outer housing of control module (512). Task light (525) is generally configured to provide light that may be movably directed towards areas proximate control module (512). The light provided may be generally unfocused or diffused to provide uniform lighting to an area. In other versions, task light (525) may be configured to adjust the focus of emitted light from a generally unfocused or diffused configuration to a bright and focused configuration. In such versions, adjustment of focus may be desirable to switch between illuminating a large area consistently and illuminating a small area with bright and focused light.

Unlike task light (425) described above, task light (525) of the present version is positioned proximate display (540). In particular, task light (525) is positioned directly above display (540). In this version, task light (525) may include a swivel feature and/or a telescoping feature to permit adjustment of the position and direction of task light (523) relative to control module (512). In some versions, such telescoping features may be configured similarly to goose neck (427), while also being configured to retract within a portion of control module (512). In other versions, task light (525) may be fixed to control module (512) such that light is emitted in a fixed direction.

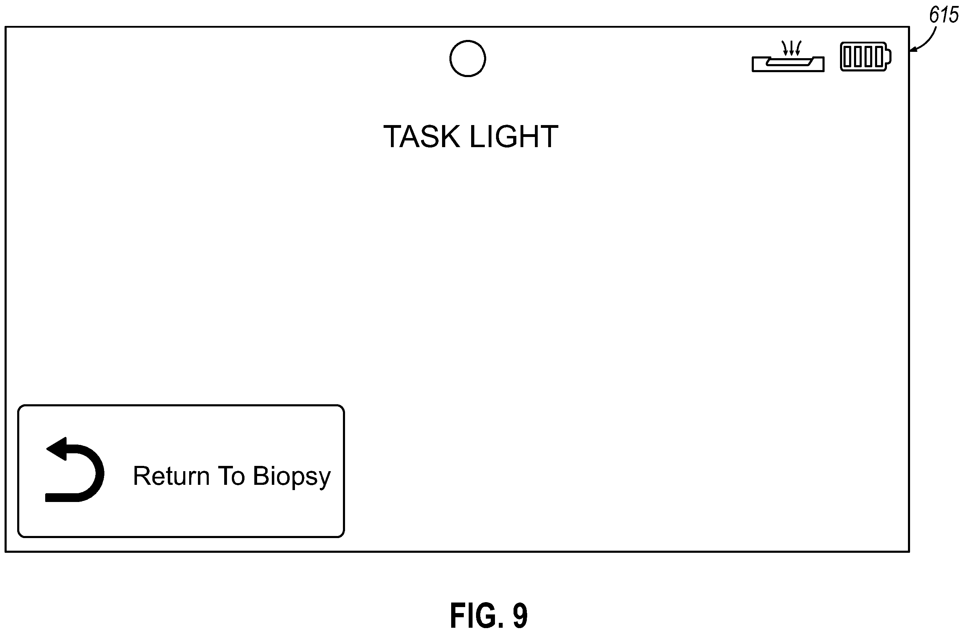

FIG. 9 shows an example of a user interface screen (615) that may be used in any one of displays (40, 240, 540) described above. As can be seen, user interface screen (615) is substantially white in color such that a substantial amount of white light may be emitted from any one of displays (40, 240, 540) described above. In other words, in some versions, light may be provided by displays (40, 240, 540) directly in addition to, or in lieu of task lights (425, 525) described above. Although not shown, it should be understood that in some versions, control modules (12, 212, 512) described herein may also be configured to increase the brightness of each respective display (40, 240, 540) to a maximum brightness level to further increase the amount of light provided by display (40, 240, 540).

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A control module for use in an MRI guided biopsy procedure using an MRI coil, the control module comprising: a body, including: one or more ports configured to couple the control module to a biopsy device, and a display configured to output one or more biopsy device status indicators; and a gauss detection assembly, the gauss detection assembly including one or more sensors integrated into the body of the control module, each sensor of the one or more sensors being configured to detect an electromagnetic field emitted from the MRI coil.

### Example 2

The control module of Example 1, the gauss detection assembly further including a detector array, each sensor of the one or more sensors being arranged on the detector array.

### Example 3

The control module of Example 2, the detector array including a single sensor

### Example 4

The control module of Example 2, the detector array including a plurality of sensors, each sensor being positioned along an axis aligned with a geometric feature of the body, each sensor being configured to detect the electromagnetic field emitted from the MRI coil in a single dimension or multiple dimensions.

### Example 5

The control module of any of Examples 1 through 4, further comprising a signal processing system, the signal processing system being in communication with each sensor of the one or more sensors, the signal processing system being configured to initiate one or more operational controls when the electromagnetic field detected by the one or more sensors exceeds a predetermined threshold.

### Example 6

The control module of Example 1, further comprising a signal processing system, the one or more sensors including a plurality of sensors, the signal processing system being in communication with each sensor of the plurality of sensors, the signal processing system being configured to: receive a detected electromagnetic field signal from each sensor of the plurality of sensors, and compute the position of the control module relative to the MRI coil.

### Example 7

The control module of Example 1, further comprising a signal processing system, the one or more sensors including a plurality of sensors, the signal processing system being in communication with each sensor of the plurality of sensors, the signal processing system being configured to: receive a detected electromagnetic field signal from each sensor of the plurality of sensors, compute the orientation of the control module relative to the MRI coil, and compare one or more of the detected electromagnetic field signals to a predetermined threshold value corresponding to the computed orientation of the control module relative to the MRI coil.

### Example 8

The control module of Example 1, further comprising a signal processing system, the one or more sensors including a plurality of sensors, the signal processing system being in communication with each sensor of the plurality of sensors, the signal processing system being configured to: receive a detected electromagnetic field signal from each sensor of the plurality of sensors, compute the orientation of the control module relative to the MRI coil, compare one or more of the detected electromagnetic field signals to a predetermined threshold value corresponding to the computed orientation of the control module relative to the MRI coil, and initiate one or more operational controls when the predetermined threshold value is exceeded by one or more of the detected electromagnetic field signals.

### Example 9

The control module of any of Examples 1 through 4, further comprising a signal processing system and an indicator, the signal processing system being in communication with each sensor of the one or more sensors, the signal processing system being further in communication with the indicator, the signal processing system being configured to initiate one or more operational controls via the indicator when the electromagnetic field detected by the one or more sensors exceeds a predetermined threshold.

### Example 10

The control module of Example 9, the indicator including a first illuminator, a second illuminator, and a third illuminator, the signal processing system being configured to illuminate each of the first illuminator, second illuminator, and third illuminator based on the electromagnetic field detected by the one or more sensors.

### Example 11

The control module of Example 9, the indicator being configured to emit a light of a predetermined color, the signal processing system being configured to emit the light of a predetermined color based on the electromagnetic field detected by the one or more sensors.

### Example 12

The control module of Example 9, the indicator including a first indicator and a second indicator, the first indicator being configured to emit a green light and to emit a yellow light, the second indicator including a buzzer, the signal processing system being configured to emit the green light or the yellow light based on the electromagnetic field detected by the one or more sensors, the signal processing system being further configured to initiate the buzzer when the electromagnetic field detected by the one or more sensors exceeds a predetermined threshold.

### Example 13

The control module of any of Example 9, the indicator including a wheel lock associated with one or more wheels attached to the body, the signal processing system being further configured to transition the wheel lock from an open position to a closed position when the electromagnetic field detected by the one or more sensors exceeds a predetermined threshold.

### Example 14

The control module of any of Examples 9 through 13, the indicator being remotely positioned relative to the body.

### Example 15

The control module of any of Examples 1 through 14, each sensor of the one or more sensors including a hall effect sensor.

### Example 16

A biopsy system for performing an MRI guided biopsy procedure within an MRI suite, the MRI suite including an MRI coil, the system comprising: a control module; a biopsy device in communication with the control module; and a gauss detection assembly, the gauss detection assembly including: a detector array, the detector array including one or more sensors permanently secured in a fixed position relative to a portion of the control module, and a signal processing system, the signal processing system being in communication with the detector array to receive a detected electromagnetic field from each sensor of the one or more sensors, the signal processing system being configured to initiate one or more operational controls based on the detected electromagnetic field communicated from the detector array.

### Example 17

The system of Example 16, the detector array being positioned proximate a top surface of the control module.

### Example 18

The system of Examples 16 or 17, the detector array including a plurality of sensors, each sensor of the plurality of sensors being positioned on a common plane, each sensor of the plurality of sensors being further positioned offset from each of the other sensors on the common plane.

### Example 19

The system of Examples 16 or 17, the detector array including a first sensor and a second sensor, the control module defining a width axis extending along the widest horizontal dimension of the control module, the first sensor and second sensor being positioned along a sensor axis, the sensor axis being parallel to the width axis.

### Example 20

The system of any of Examples 16 through 19, the control module including a display, the signal processing system being in communication with the display, the signal processing system being configured to drive one or more alerts on the display based on the detected electromagnetic field.

### Example 21

The system of any of Examples 16 through 20, the biopsy device including an indicator, the signal processing system being in communication with the indicator, the signal processing system being configured to drive illumination of one or more features of the indicator based on the detected electromagnetic field communicated from the detector array.

### Example 22

A method of detecting the presence of an electromagnetic field generated by an MRI coil relative to a control module of a MRI biopsy system, comprising: moving the control module relative to the MRI coil; detecting the electromagnetic field generated by the MRI coil using a detector array integrated into the control module to generate a detected electromagnetic field signal; comparing the detected electromagnetic field signal to one or more threshold values; and initiating one or more operational controls when the detected electromagnetic field signal exceeds at least one threshold value of the one or more threshold values.

### Example 23

The method of Example 22, the step of detecting the electromagnetic field generated by the MRI coil including generating a first detected electromagnetic field signal and a second detected electromagnetic field signal, the first detected electromagnetic field signal corresponding to a first sensor of the detector array, the second detected electromagnetic field signal corresponding to a second sensor of the detector array.

### Example 24

The method of Example 22, further comprising computing a spatial position of the control module relative to the MRI coil based on the first detected electromagnetic field signal and a second detected electromagnetic field signal.

### Example 25

The method of Example 24, the one or more threshold values including a plurality of threshold values, each threshold value of the plurality of threshold values corresponding to a particular spatial position of the control module, the method further comprising selecting a particular threshold value from the plurality of threshold values based on the spatial position of the control module computed based on the first detected electromagnetic field signal and a second detected electromagnetic field signal.

### Example 26

The method of any of Examples 22 through 25, the step of initiating the one or more operational controls including illuminating one or more lights of an indicator.

### Example 27

The method of any of Examples 22 through 26, the step of initiating the one or more operational controls including emitting one or more tones or generating one or more vibrational patterns.

### Example 28

The method of any of Examples 22 through 27, the step of initiating the one or more operational controls including locking movement of one or more wheels of the control module.

### Example 29

The method of any of Examples 22 through 28, the step of initiating the one or more operational controls including disabling one or more functions of the control module.

### VI. Conclusion

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A control module for use in an MRI guided biopsy procedure using an MRI coil, the control module comprising:
(a) a body, including:
(i) one or more ports configured to couple the control module to a biopsy device, and
(ii) a display configured to output one or more biopsy device status indicators; and
(b) a gauss detection assembly, the gauss detection assembly including one or more sensors integrated into the body of the control module, each sensor of the one or more sensors being configured to detect an electromagnetic field emitted from the MRI coil.

2. The control module of claim 1, the gauss detection assembly further including a detector array, each sensor of the one or more sensors being arranged on the detector array.

3. The control module of claim 2, the detector array including a single sensor.

4. The control module of claim 2, the detector array including a plurality of sensors, each sensor being positioned along an axis aligned with a geometric feature of the body, each sensor being configured to detect the electromagnetic field emitted from the MRI coil in a single dimension or multiple dimensions.

5. The control module of any of claims 1 through 4, further comprising a signal processing system, the signal processing system being in communication with each sensor of the one or more sensors, the signal processing system being configured to initiate one or more operational controls when the electromagnetic field detected by the one or more sensors exceeds a predetermined threshold.

6. The control module of claim 1, further comprising a signal processing system, the one or more sensors including a plurality of sensors, the signal processing system being in communication with each sensor of the plurality of sensors, the signal processing system being configured to:
receive a detected electromagnetic field signal from each sensor of the plurality of sensors, and
compute the position of the control module relative to the MRI coil.

7. The control module of claim 1, further comprising a signal processing system, the one or more sensors including a plurality of sensors, the signal processing system being in communication with each sensor of the plurality of sensors, the signal processing system being configured to:
receive a detected electromagnetic field signal from each sensor of the plurality of sensors,
compute the orientation of the control module relative to the MRI coil, and
compare one or more of the detected electromagnetic field signals to a predetermined threshold value corresponding to the computed orientation of the control module relative to the MRI coil.

8. The control module of claim 1, further comprising a signal processing system, the one or more sensors including a plurality of sensors, the signal processing system being in communication with each sensor of the plurality of sensors, the signal processing system being configured to:
receive a detected electromagnetic field signal from each sensor of the plurality of sensors,
compute the orientation of the control module relative to the MRI coil,
compare one or more of the detected electromagnetic field signals to a predetermined threshold value corresponding to the computed orientation of the control module relative to the MRI coil, and
initiate one or more operational controls when the predetermined threshold value is exceeded by one or more of the detected electromagnetic field signals.

9. The control module of any of claims 1 through 4, further comprising a signal processing system and an indicator, the signal processing system being in communication with each sensor of the one or more sensors, the signal processing system being further in communication with the indicator, the signal processing system being configured to initiate one or more operational controls via the indicator when the electromagnetic field detected by the one or more sensors exceeds a predetermined threshold.

10. The control module of claim 9, the indicator including a first illuminator, a second illuminator, and a third illuminator, the signal processing system being configured to illuminate each of the first illuminator, second illuminator, and third illuminator based on the electromagnetic field detected by the one or more sensors.

11. The control module of claims 9, the indicator being configured to emit a light of a predetermined color, the signal processing system being configured to the light of the predetermined color based on the electromagnetic field detected by the one or more sensors.

12. The control module of claim 9, the indicator including a first indicator and a second indicator, the first indicator being configured to emit a green light and to emit a yellow light, the second indicator including a buzzer, the signal processing system being configured to emit the green light or the yellow light based on the electromagnetic field detected by the one or more sensors, the signal processing system being further configured to initiate the buzzer when the electromagnetic field detected by the one or more sensors exceeds a predetermined threshold.

13. The control module of any of claims 9, the indicator including a wheel lock associated with one or more wheels attached to the body, the signal processing system being further configured to transition the wheel lock from an open position to a closed position when the electromagnetic field detected by the one or more sensors exceeds a predetermined threshold.

14. The control module of any of claims 9 through 13, the indicator being remotely positioned relative to the body.

15. The control module of any of claims 1 through 14, each sensor of the one or more sensors including a hall effect sensor.
